# EUROPÄISCHE PATENTSCHRIFT

(11) **EP 1 419 738 B1**
(45) Veröffentlichungstag und Bekanntmachung des Hinweises auf die Patenterteilung: **15.02.2006**
(21) Anmeldenummer: 03026251.3
(22) Anmeldetag: 14.11.2003
(51) Int. Cl.: A61B 10/00

(54) **Gerät zur Schwangerschaftsverhütung und Familienplanung**
Device for prevention of pregnancy and fertility control
Outil pour la prévention de la grossesse et pour la régulation de la fertilité

(30) Priorität: 14.11.2002 DE 10253335
(43) Veröffentlichungstag der Anmeldung: 19.05.2004
(73) Patentinhaber: oneworld lifestyle Products AG, 6300 Zug (CH)
(72) Erfinder: Heitz, Kilian, 8906 Bonstetten (CH)
(74) Vertreter: Frohwitter, Bernhard

(56) Entgegenhaltungen:
- WO-A-01/19251
- CA-A- 2 290 898
- US-A- 4 771 791
- US-A- 5 837 197
- US-A1- 2002 111 561

## Beschreibung

Zur Schwangerschaftsverhütung werden bisher verschiedene Methoden entwickelt und angewandt. Die am weitesten verbreiteten Methoden sind mechanischer, wie Kondom oder Spirale, hormoneller (Pille) und chemischer Natur, wie z.B. Zäpfchen, Creme, Schaum. Sowohl für die Schwangerschaftsverhütung als auch für die Familienplanung wurden in der letzteren Zeit Vorrichtungen entwickelt, die in der Lage sind die Wahrscheinlichkeit der Empfängnis an einem bestimmten Tag zu beurteilen. Die Fruchtbarkeit einer Frau ändert sich zyklisch, so dass etwa alle 28 Tage eine Eizelle so weit heranreift, dass sie sich von dem Eierstock ablöst und befruchtet werden kann. Das Ereignis des Ablösens wird als Eisprung oder Ovulation bezeichnet. Die Eizelle ist nach der Ovulation nur etwa 12 bis höchstens 24 Stunden befruchtungsfähig. Dagegen sind Samenzellen im Durchschnitt 72 Stunden, in Ausnahmefällen sogar bis zu fünf Tage im Körper der Frau lebensfähig. Der Zeitraum der höchsten Fruchtbarkeit beginnt deshalb etwa 72 Stunden vor der Ovulation und endet etwa 12 Stunden danach. Alle bekannten Methoden, die die Fruchtbarkeitsphase bestimmen, versuchen daher den Zeitpunkt des Eisprungs vorherzubestimmen, da die Wahrscheinlichkeit der Empfängnis um den Eisprung am größten ist. Innerhalb eines Zyklus variieren viele Parameter, wie z.B. Körpertemperatur bzw. Basaltemperatur, LH-Hormonspiegel, die Beschaffenheit und die Menge des Zervixschleims abhängig von der Phase des Zyklus. Anhand dieser Parameter ist es theoretisch möglich, Methoden zu entwickeln, die es erlauben, den Zeitpunkt des Eisprungs und damit die fruchtbaren Tage einer Frau zu bestimmen.

Es ist bekannt, dass der weibliche Zyklus einer komplizierten hormonellen Steuerung unterworfen ist. Die Dauer des Zyklus beträgt im Durchschnitt 28 Tage, variiert jedoch zwischen 24 und 32 Tagen oder tritt unregelmäßig auf. Eine bekannte Methode zur Bestimmung des Fruchtbarkeitszeitraums ist die sog. Kalendermethode, die annimmt, dass der Eisprung regelmäßig in der Mitte des Zyklus stattfindet. Es wird dabei vorausgesetzt, dass die Dauer des Zyklus 28 Tage beträgt und der Eisprung 14 Tage nach dem ersten Tag des letzten Zyklus stattfindet. Da jedoch jeder Zyklus gewissen Schwankungen unterworfen ist, ist die Kalendermethode ungenau und daher für eine zuverlässige Schwangerschaftsverhütung bzw. Familienplanung eher ungeeignet. WO 01/36212 beschreibt eine Vorrichtung, die ausschließlich auf der Kalendermethode basiert und darüber den Eisprung und damit die Fruchtbarkeit einer Frau bestimmt.

Daneben ist die Messung der Basaltemperatur eine weitverbreitete Methode zur Bestimmung des Eisprungs. Nach jedem Eisprung wird das Geschlechtshormon Gestagen gebildet, das die Eigenschaft hat, auf das Wärmeregulationszentrum einzuwirken, wodurch die Körpertemperatur um etwa 0,4 bis 0,6°C ansteigt. Das hat zur Folge, dass die Durchschnittstemperatur der ersten Zyklushälfte (vor dem Eisprung) niedriger ist als die Durchschnittstemperatur der zweiten Zyklushälfte (nach dem Eisprung). Da das Gestagen nur gebildet wird, wenn ein Eisprung vorausgegangen ist, kann der Temperaturanstieg als ein sicheres Zeichen für einen Eisprung gewertet werden. Liegen jedoch Faktoren wie z.B. eine Erkältung oder Migräne vor, können diese ebenso zu einer Erhöhung der Körpertemperatur führen und eine Bestimmung des Eisprungs wird damit schwierig bzw. sogar unmöglich. Dies zeigt, dass die Anwendung der Basaltemperaturmethode geringe Genauigkeit aufweist und keine Voraussage des Eisprungs erlaubt.

EP 0 195 207 beschreibt eine Vorrichtung zur Empfängnisregulierung, mit einer Temperaturmesseinrichtung zur Messung der Körpertemperatur, wobei die Temperaturmesseinrichtung in einem in den Körper einführbaren Gehäuse untergebracht ist, das außerdem einen Datenspeicher enthält, der unter Steuerung durch einen Zeitgeber in festgelegten Zeitintervallen die Temperatur speichert und dass eine Sende- und Empfangseinrichtung vorgesehen ist, die auf ein externes Befehlsignal hin Daten aus dem Datenspeicher aussendet. WO87/02876 beschreibt eine Vorrichtung zur Bestimmung der Basaltemperaturkurve und zur Ermittlung der Konzeptionstage, mit einer Einrichtung zur Messung der Körperkerntemperatur, einer Auswerteeinheit, in der die täglich gemessenen Körperkemtemperaturwerte in Zuordnung zum Messdatum speicherbar und auswertbar sind, und einer Anzeige- und Bedienungseinheit, sowie gegebenenfalls einer Uhr- und Weckfunktion, wobei die Auswerteeinheit zur Ermittlung des Eisprungs zunächst eine Reihe gemessener, aufeinanderfolgender Temperaturwerte, deren Zahl vorgebbar ist, mit einem Temperatur-Startwert vergleicht, den sie aus der zu Beginn des Zyklus gemessenen Temperaturwert-Reihe derart bestimmt hat, dass mehr als die Hälfte der zu Beginn des Zyklus gemessenen Temperaturwerte um wenigstens 0,1°C kleiner als der Temperatur-Startwert ist, und dass die Auswerteinheit dann, wenn mehr als die Hälfte der zuletzt gemessenen Temperaturwerte dieser Reihe größer als der Startwert ist, mit einem um 0,1°C gegenüber dem Startwert erhöhten zweiten Temperaturwert überprüft, bei welcher Reihe von Temperaturwerten, die vom Zyklusbeginn zum Zyklusende bzw. zum aktuellen Messtag hin verschoben wird, zum ersten Mal mehr als die Hälfte der gemessenen Temperaturwerte gleich groß oder größer als der zweite Temperaturwert ist, und den Tag der Messung des ersten Temperaturwertes dieser Reihe, der sich mindestens auf dem Niveau des zweiten Temperaturwertes befindet, als Mittelpunkt einer neuen Reihe bestimmt, in der das Minimum berechnet wird, das den Ovulationstag darstellt, wobei beim Auftreten mehrerer Minima das dem Zyklusende nächst gilt.

Eine andere Methode zur Bestimmung des Eisprungs ist die Zervix-Schleimmethode, die von Prof. Billing entwickelt worden ist. Zu Beginn des Zyklus, nach der Menstruation, tritt wenig Zervixschleim aus. Um die Zeit des Eisprunges (Ovulation) produzieren die Zervixdrüsen Schleim, der anfangs dick und in den folgenden Tagen zunehmend klarer und flüssiger wird. Es wird angenommen, dass die fruchtbaren Tage mit dem Auftreten des Schleimes beginnen und mehrere Tage dauem. Danach wird der Schleim wieder zähflüssiger und der Fruchtbarkeitszeitraum ist vorüber. Durch die Anwendung chemischer Verhütungsmittel oder durch andere Umstände, kann aber die natürliche Beschaffenheit des Zervixschleimes verändert werden. Neben dem Zervixschleim verändert sich der Speichel während des Zyklus, so dass auch aufgrund dieser Veränderung eine Aussage getroffen werden kann, ob der Eisprung stattgefunden hat. WO 01/06932 beschreibt eine Vorrichtung, durch die der Speichel oder der Zervixschleim untersucht werden können, um den Fruchtbarkeitszeitraum zu bestimmen.

WO O1/19251 A1 beschreibt eine Vorrichtung, ein "Biometer", zur Messung und Anzeige der Dauer von biologischen Funktionen wie etwa die Dauer der Ovulation. Somit eignet sich diese Vorrichtung zur Schwangerschaftsverhütung und Familienplanung. Gespeichert werden weiter Körpertemperatur, Fruchtbarkeitsstatus, prämenstruelle Symptome, und/oder Dauer der Schwangerschaft. Die Daten werden auf einem Display angezeigt. Eine Gewichtung der Daten erfolgt nicht.

Der Eisprung kann auch durch die Bestimmung der Konzentration des luteinisierenden Hormons (LH) im Urin festgestellt werden und es gibt eine Vielzahl von Vorrichtungen auf dem Markt, die diese Methode verwenden. Um den Zeitpunkt des Eisprung kommt es zu einem Anstieg des LH, der ein gutes Indiz für den Zeitpunkt des Eisprungs ist. Die Vorrichtung zur Bestimmung der LH-Konzentration beinhaltet Teststreifen mit monoklonalen Antikörpern, die für LH spezifisch sind. Die anschließende ELISA oder EMIT-Methode erlaubt aufgrund einer Farbänderung die Konzentrationsbestimmung von LH. Durch die tägliche Anwendung dieser Methode kann festgestellt werden, ob die Konzentration von LH von einem auf den anderen Tag ansteigt. EP 0 383 619 beschreibt ein Kit mit den Teststreifen zur Bestimmung der Konzentration von LH im Urin. Diese Methode ist deutlich zuverlässiger als die zuerst genannten Methoden, jedoch ist die Anwendung aufwendig und die Teststreifen sind teuer.

Alle oben angesprochenen Methoden können verwendet werden, um zu bestimmen, ob ein Eisprung stattgefunden hat. Keine dieser Methoden alleine kann aber den Zeitpunkt des Eisprungs exakt voraussagen. Deshalb beinhalten die auf dem Markt vorhandenen Geräte eine oder mehrere Vorrichtungen, die die Durchführung mehrerer Methoden erlaubt, um eine Voraussage zu treffen, wann der Zeitpunkt des nächsten Eisprungs sein wird. Die Sicherheit einer Verhütungsmethode wird mittels des Pearl Index wiedergegeben, wobei die meisten Geräte auf dem Markt einen Wert zwischen 0,5 und 7 auf dem Pearl-Index aufweisen (je niedriger der Wert desto sicherer ist das Gerät). Die Genauigkeit der vorhandnen Geräte schwankt abhängig von der verwendeten Messmethode. Das Problem ist, dass die Methoden entweder niedrige Sicherheit aufweisen oder aber vergleichsweise kompliziert in der Anwendung sind, wenn größere Genauigkeit gewünscht wird. Der vorliegenden Erfindung liegt die Aufgabe zugrunde, ein Gerät bereitzustellen, das einfache Handhabung mit großer Genauigkeit bei der Voraussage und Bestimmung des Eisprungs verbindet und dabei keine teueren Komponenten wie z.B. Teststreifen mit monoklonalen Antikörpern erfordert. Die Aufgabe der vorliegenden Erfindung ist es ein solches Gerät bereitzustellen.

Das erfindungsgemäße Gerät kombiniert folgende Methoden, um den voraussichtlichen Zeitpunkt des Eisprungs zu bestimmen: Die Kalendermethode, die Basaltemperaturmethode und die Speichelmethode.

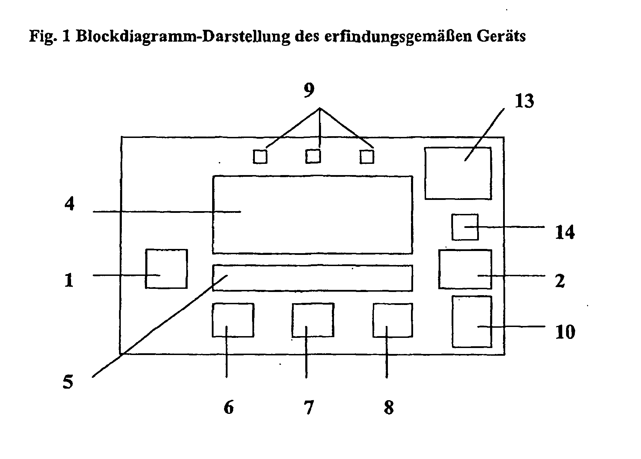

Die Lösung der Aufgabe ist gemäß dem Anspruch 1 wiedergegeben. Die Ausführungsform gemäß Anspruch 1 stellt ein Gerät zur Familienplanung und/oder Verhütung bereit, mit einem Mittel (1) zur Bestimmung der Basaltemperatur, einem Mittel (7) zur Eingabe des ersten Tages des Zyklus, einem Mittel (8) zur Eirigabe der Beschaffenheit des Speichels, einer Vorrichtung zur Verarbeitung der durch die Mittel (1, 7, 8) bereitgestellten Daten (13), einer Speichervorrichtung zum Speichern von zumindest einigen der bereitgestellten Daten (14) und einer Anzeigeeinrichtung, wobei die Vorrichtung zur Verarbeitung der Daten derart ausgestaltet ist, dass in Abhängigkeit von zumindest einigen der bereitgestellten Daten eines jeden Mittels eine Aussage zur Fruchtbarkeit an einem beliebigen Tag ermittelt wird, die über die Anzeigeeinrichtung anzeigbar ist und die Daten beim Ermitteln der Aussage zur Fruchtbarkeit unterschiedlich gewichtet werden (siehe Figur 1).

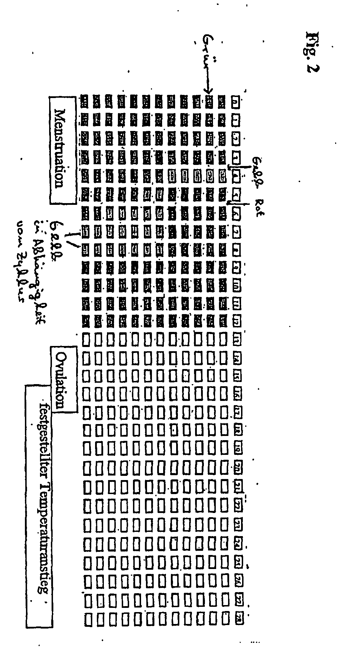

Beispielsweise können ausschließlich Daten zur Basaltemperatur während des ersten bzw. ersten und zweiten Zyklus berücksichtigt werden, um eine möglichst große Sicherheit hinsichtlich einer Aussage zur Fruchtbarkeit an einem beliebigen Tag im Anfangsstadium der Benutzung des erfindungsgemäßen Geräts zu erzielen. In einer bevorzugten Ausführungsform zeigt die Anzeigeeinrichtung zu Beginn des Gebrauchs Fixwerte an, zum Beispiel vier mögliche unfruchtbare Tage, gefolgt von einem Tag, an dem eine Aussage zur möglichen Fruchtbarkeit bzw. Unfruchtbarkeit nicht möglich ist, gefolgt von einer Anzahl möglicher fruchtbarer Tage (siehe Figur 2).

In einer weiteren bevorzugten Ausführungsform werden bis zum einschließlich fünften Zyklus in der Zeitspanne vor der Ovulation Fixwerte verwendet. Erst ab dem sechsten Zyklus wird die Regelmäßigkeit des Zyklusbeginns (Eingabe des ersten Tages der Periode) in einer Aussage zur Fruchtbarkeit an einem beliebigen Tag berücksichtigt, zum Beispiel dadurch, dass von dem zu erwartenden Eisprung je nach Regelmäßigkeit des Zyklusbeginns mindestens sechs Tage zurückgerechnet wird.

In einer weiteren bevorzugten Ausführungsform werden zusätzlich zu den Fixwerten in der Zeitspanne vor der Ovulation Fixwerte in der Zeitspanne nach erfolgter Ovulation verwendet, zum Beispiel zwei Tage (Ovulation plus 1 Tag). Dies könnte beispielsweise dazu führen, dass die Aussage zur Fruchtbarkeit an mindestens acht aufeinanderfolgenden Tagen einen möglichen fruchtbaren Tag ergibt.

Ab dem zweiten Zyklus können zusätzlich Daten zur Beschaffenheit des Speichels berücksichtigt werden. Eine Eingabe von Daten zur Beschaffenheit des Speichels können aber auch lediglich zur Gewöhnung der Benutzerin dienen, ohne dass sie Einfluss auf die Auswertung in dem laufenden Monat haben. In einer weiteren Ausführungsform hat die Speichelmessung ab dem dritten Zyklus einen Einfluss auf Aussage zur Fruchtbarkeit an einem beliebigen Tag, so dass beispielsweise die Anzahl der möglichen fruchtbaren Tagen nach der Ovulation abnehmen könnte.

Die Vorrichtung gemäß Anspruch 1 ist auch dadurch gekennzeichnet, dass das Gerät zur Verarbeitung der Daten derart ausgestaltet ist, dass die Daten beim Ermitteln der Aussage zur Fruchtbarkeit unterschiedlich gewichtet werden, wobei die Gewichtung der Daten in Abhängigkeit der vormals bereitgestellten, gespeicherten Daten variieren können.

Gemäß einer besonderen Ausführungsform ist das erfindungsgemäße Gerät dadurch gekennzeichnet, dass die Aussage zur Fruchtbarkeit in stärkerem Maße von den durch das Mittel (1) zur Bestimmung der Basaltemperatur bereitgestellten Daten abhängt, als von den durch das Mittel (7) zur Eingabe des ersten Tages des Zyklus bereitgestellten Daten und dass die durch das Mittel (7) zur Eingabe des ersten Tages des Zyklus bereitgestellten Daten ihrerseits einen größeren Einfluss auf die Aussage zur Fruchtbarkeit haben, als die durch das Mittel (8) zur Eingabe der Beschaffenheit des Speichels bereitgestellten Daten.

Gemäß einer weiteren besonderen Ausführungsform ist das erfindungsgemäße Gerät dadurch gekennzeichnet, dass die Gewichtung der durch das Mittel (1) zur Bestimmung der Basaltemperatur bereitgestellten Daten vorzugsweise bei 50-90%, besonders bevorzugt bei 60-80%, die Gewichtung der durch das Mittel (7) zur Eingabe des ersten Tages des Zyklus bereitgestellten Daten, vorzugsweise bei 5-35%, besonders bevorzugt bei 10-30%, und die Gewichtung der durch das Mittel (8) zur Eingabe der Beschaffenheit des Speichels bereitgestellten Daten vorzugsweise bei 2-20%, besonders bevorzugt bei 5-15%, liegt.

Die Kalendermethode ist gemäß dieser besonderen Ausführungsform weniger gewichtet als die Temperaturmethode, da die Kalendermethode wegen der natürlichen Zyklusschwankungen weniger zuverlässig ist als die Temperaturmethode. Die Gewichtung der Speicheltestmethode ist niedriger als die Gewichtung der Kalendermethode, da auch wenn die Kalendermethode nicht sehr zuverlässig ist, die Kalendermethode immerhin eine objektive Methode ist, während die Speicheltestmethode sehr subjektiv ist.

Aufgrund der Kombination dreier Methoden ist das erfindungsgemäße Gerät in der Lage, die Temperaturschwankungen, die nicht durch den Zyklus hervorgerufen sind, sondern auf anderen Faktoren wie einer Erkältung oder Migräne beruhen, zu erkennen und daraufhin diese Daten aufgrund der Temperaturschwankungen bei der Bestimmung der möglichen fruchtbaren bzw. unfruchtbaren Tagen zu verwerfen.

Eine Besonderheit des erfindungsgemäßen Geräts ist, dass nicht alle Messungen jeden Tag durchgerührt werden müssen. In einer besonderen Ausführungsform ist das Gerät dadurch gekennzeichnet, dass es eine Zeitmessvorrichtung und eine Aufforderungseinrichtung zur Aufforderung einer Benutzerin zur Eingabe von Daten nach Verstreichen einer vorbestimmten Zeitspanne umfasst. Bei einer weiteren Ausführungsform verlangt das erfindungsgemäße Gerät mittels einer Aufforderungseinrichtung die Eingabe über die Menge und die Beschaffenheit des Speichels vorzugsweise 5-10 mal, besonders bevorzugt 6-8 mal pro Zyklus.

In einer weiteren Ausführungsform ist das Gerät selbständig in der Lage einen Zyklus und insbesondere die Dauer bzw. den Verlauf des Zyklus zu erkennen. Die maximale Genauigkeit ist daher vorzugsweise vor dem sechsten oder siebten Zyklus, besonders bevorzugt vor dem fünften Zyklus erreicht.

Die Vorteile des erfindungsgemäßen Geräts liegen dabei in seiner einfachen Handhabung, an seinen klaren, unmissverständlichen Anzeigen und Aufforderungen und an der Steuerung durch einen modernen Microprozessor.

Des Weiteren umfasst die vorliegende Erfindung die Verwendungen und ein Verfahren zur Anwendung des erfindungsgemäßen Geräts.

Die Vorrichtung wird zur Bestimmung der Ovulationsphase mittels Kalender-, Basaltemperatur- und Speichelmethode verwendet, deren Werte gegeneinander verglichen werden. Dabei wird die Vorrichtung zur Familienplanung, d.h. zur Schwangerschaftsverhütung und/oder zur Erfüllung eines Kinderwunsches eingesetzt. Die Vorrichtung wird dabei vorzugsweise mehrmals pro Woche, besonders bevorzugt einmal täglich verwendet.

Das Gerät ist zur Verarbeitung der Daten derart ausgestaltet, dass die Aussage zur Fruchtbarkeit zwischen möglichen fruchtbaren und unfruchtbaren Tagen unterscheidet und dass die möglichen fruchtbaren und unfruchtbaren Tagen unterschiedlich optisch dargestellt werden, wobei bevorzugt die Anzeigeeinrichtung die möglichen fruchtbaren Tage in roter Farbe und die möglichen unfruchtbaren Tage in grüner Farbe signalisiert.

Das Gerät ist weiterhin dadurch gekennzeichnet, dass in der Aussage zur Fruchtbarkeit auch sogenannte Übergangstage vorgesehen sind, an denen nicht zwischen möglichen fruchtbaren und unfruchtbaren Tagen unterschieden wird, wobei die Übergangstage von den möglichen fruchtbaren und unfruchtbaren Tagen optisch unterschiedlich dargestellt werden. In einer bevorzugten Ausführungsform signalisiert die Anzeigevorrichtung die Übergangstage in gelber Farbe.

In einer weiteren Ausführungsform ist das Gerät durch ein Mittel zum Erkennen eines Temperaturanstieges aus den durch das Mittel (1) zur Bestimmung der Basaltemperatur bereitgestellten Daten gekennzeichnet, wobei beim Ermitteln der Aussage zur Fruchtbarkeit ein erster Zyklusabschnitt zu Beginn eines Zyklus und ein zweiter Zyklusabschnitt, der nach dem erkannten Temperaturanstieg liegt, unterschieden wird.

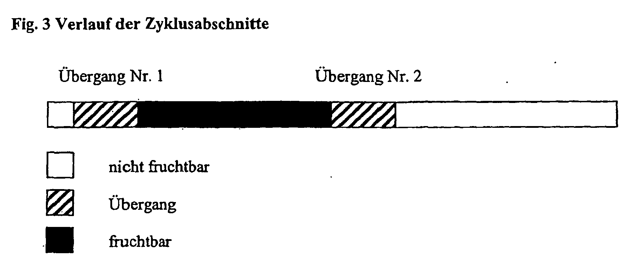

Der Begriff "erster Zyklusabschnitt" bzw. "zweiter Zyklusabschnitt" wird dahingehend verstanden, dass der erste Zyklusabschnitt die erste Zeitspanne vom Zyklusbeginn darstellt. Dieser erste Zyklusabschnitt umfasst dabei mögliche unfruchtbare Tage und den Übergang zu möglichen fruchtbaren Tagen. Der zweite Zyklusabschnitt umfasst den Übergang von möglichen fruchtbaren Tagen zu möglichen unfruchtbaren Tagen nach erfolgtem Temperaturanstieg (siehe Figur 3).

In einer weiteren Ausführungsform des Gerätes werden bis zu einem ersten Zeitpunkt, beispielsweise dem Ende des zweiten durch das Gerät registrierten Zyklus, in der Aussage zur Fruchtbarkeit im zweiten Zyklusabschnitt die Anzahl der möglichen fruchtbaren Tage vorgegeben. Dabei kann nach dem ersten Zeitpunkt in der Aussage zur Fruchtbarkeit im zweiten Zyklusabschnitt die Anzahl von möglichen fruchtbaren Tagen in Abhängigkeit einer Auswertung gespeicherter Daten bestimmt werden.

In einer bevorzugten Ausführungsform ist das Gerät dadurch gekennzeichnet, dass aus den gespeicherten Daten ein Indikator für die Regelmäßigkeit der Zyklusdauer bzw. des Zyklusverlaufs abgeleitet wird und dass in der Aussage zur Fruchtbarkeit im zweiten Zyklusabschnitt die Anzahl der möglichen unfruchtbaren Tagen in Abhängigkeit dieses Indikator und/oder der Anzahl der durch das Gerät registrierten Zyklen bestimmt wird.

Der Indikator gemäß der vorliegenden Erfindung ist beispielsweise die Standartabweichung (Sigma) des Tages für den Tag der Ovulation (Temperaturanstieg). Dabei wird der Tag, an der die Ovulation des nächsten Zyklus erfolgte, in Relation zu dem Mittelwert der Tage gesetzt, an denen die Ovulation der vorhergehenden Zyklen erfolgte (siehe Tabelle 1). "Gut" bedeutet ein Sigma von 0.8 vom Mittelwert. "Schlecht" bedeutet ein Sigma von mehr als 2.0 und "Normal" bedeutet ein Sigma zwischen 0,8 und 2,0. Bevorzugt erfolgt eine Berechnung des Sigmas nach dem 4. Zyklus. Die Anzahl der gespeicherten Tage, an denen die Ovulation erfolgte, ist unbegrenzt. Bevorzugt wird eine Speicherung der letzten 6 bis 24 Zyklen, besonders bevorzugt der letzten 15 Zyklen.

In einer weiteren Ausführungsform werden bei der Aussage zur Fruchtbarkeit im zweiten Zyklusabschnitt die Anzahl von möglichen fruchtbaren Tagen in Abhängigkeit der durch das Mittel (8) zur Eingabe der Beschaffenheit des Speichels bereitgestellten Daten bestimmt bzw. beeinflusst.

In einer weiteren Ausführungsform ist das erfindungsgemäße Gerät dadurch gekennzeichnet, dass bis zu einem zweiten Zeitpunkt, beispielsweise dem Ende des fünften durch das Gerät registrierten Zyklus, in der Aussage zur Fruchtbarkeit im ersten Zyklusabschnitt die Anzahl der möglichen unfruchtbaren Tage vorgegeben ist, wobei bevorzugt nach dem zweiten Zeitpunkt in der Aussage zur Fruchtbarkeit im ersten Zyklusabschnitt die Anzahl der möglichen unfruchtbaren Tagen in Abhängigkeit einer Auswertung gespeicherter Daten bestimmt wird.

In einer besonders bevorzugten Ausführungsform ist das erfindungsgemäße Gerät dadurch gekennzeichnet, dass aus den gespeicherten Daten ein Qualitätsfaktor abgeleitet wird und dass nach dem zweiten Zeitpunkt in der Aussage zur Fruchtbarkeit im ersten Periodenabschnitt die Anzahl von möglichen unfruchtbaren Tagen in Abhängigkeit des Qualitätsfaktors und/oder der Anzahl der durch das Gerät registrierter Perioden bestimmt wird in Abhängigkeit zunächst einiger Daten des Mittels (7) zur Eingabe des ersten Tages des Zyklus.

Der Qualitätsfaktor wird vom Computerprogramm der erfindungsgemäßen Vorrichtung berechnet (ixP2sigma) und ist ein Indiz für die Konstanz des Ovulationszeitpunkts in allen gemessenen Zyklen im Bereich von 0 bis 7. Erfolgt beispielsweise die Ovulation stets am 14. Tag, so liegt ein sehr hoher Qualitätsfaktor vor, d.h. der Qualitätsfaktor = 7 (Figur 2).

0 ≤ ixP2sigma < 0,5 ergibt einen Qualitätsfaktor von 7. 0,5 ≤ ixP2sigma < 1,0 ergibt einen Qualitätsfaktor von 6 usw. Ein niedriger Qualitätsfaktor ist ein Indiz für eine starke Variation des Ovulationszeitpunkts. 3,0 ≤ ixP2sigma < 3,5 ergibt einen Qualitätsfaktor von 1 und 3,5 ≤ ixP2sigma ergibt einen Qualitätsfaktor von 0.

Das Verfahren wird zur Bestimmung der Ovulationsphase bzw. zur Familienplanung angewendet. Dazu werden mittels Kalender-, Basaltemperatur- und Speichelmethode Parameter des weiblichen Organismus gemessen und anschließend die Werte gegeneinander verglichen. Bevorzugt gemessene Parameter sind dabei Körpertemperatur und Konsistenz des Speichels, wobei die Messung der Körpertemperatur bevorzugt oral und die Messung der Konsistenz des Speichels bevorzugt extrakorporal erfolgt. Diese Daten werden schließlich gespeichert, vorzugsweise mittels eines Mikroprozessors.

Die Erfindung wird anhand der Figuren 1 bis 5 und der Tabelle 1 näher erläutert.
Es zeigt:
Fig. 1 die Blockdiagramm-Darstellung des erfindungsgemäßen Geräts
Fig. 2 den Übergang Nr. 1 während der Anwendung über mehrere Zyklen
Fig. 3 den Verlauf der Zyklusabschnitte in der Anzeigevorrichtung
Fig. 4 die Aufsicht des erfindungsgemäßen Geräts
Fig. 5 die Rückseite des erfindungsgemäßen Geräts.

Tabelle 1 den Übergang Nr. 2

**Fig. 1** zeigt die Blockdiagramm-Darstellung des erfindungsgemäßen Geräts

**Fig. 2** zeigt den Übergang Nr. 1 während der Anwendung über mehrere Zyklen. Übergang Nr. 1 ist der erste Teil des Zyklus zu Zyklusbeginn (Blutung soeben erfolgt). Die Verwenderin ist unfruchtbar für drei Tage, so dass die Anzeigeeinrichtung vier Tage "möglicherweise unfruchtbar" anzeigt einschließlich des Tages Null (vor der erster Temperaturmessung). Ab dem vierten Tag erfolgt ein Übergang der Anzeige "möglicherweise unfruchtbar" im Zeitraum von mehreren Tagen zur Anzeige "möglicherweise fruchtbar" in Abhängigkeit davon, wann ein Übergang zu "möglicherweise fruchtbar" erfolgen muss, um eine möglichst sichere Aussage zur Fruchtbarkeit zumachen, zum Beispiel wenn das Gerät zur Schwangerschaftsverhütung verwendet wird. Die Verarbeitung der Daten erfolgt in Hinblick auf eine größt mögliche Sicherheit. Es wird angenommen, dass Sperma eine Überlebenszeit von fünf Tagen besitzt und das eine Eizelle bis zu zwei Tage nach der Ovulation befruchtbar bleibt. Es werden Daten des Zyklusbeginns von bisherigen Zyklen statistisch verwendet, um zu bestimmen, wann eine Änderung zu "möglicherweise fruchtbar" erfolgen muß. Der Qualitätsfaktor wird vom Computerprogramm der erfindungsgemäßen Vorrichtung berechnet (ixP2sigma) und ist ein Indiz für die Konstanz des Ovulationszeitpunkts in allen gemessenen Zyklen im Bereich von 0 bis 7. Erfolgt beispielsweise die Ovulation stets am 14. Tag, so liegt ein sehr hoher Qualitätsfaktor vor, d.h. der Qualitätsfaktor = 7.

### Einfluß des Zyklus:

- Zyklus # 1 bis 5: Schaltet auf Gelb (Übergang) ab dem 4. Tag
- Zyklus # 6 bis 7: Schaltet auf Gelb 8 bis 11 Tage vor dem frühesten zweiten Tag der bisher gemessenen Temperaturanstiege in Abhängigkeit von dem Qualitätsfaktor (siehe Tabelle unten)
- Zyklus # 8 bis 10: Schaltet auf Gelb 8 bis 10 Tage vor dem frühesten zweiten Tag der bisher gemessenen Temperaturanstiege in Abhängigkeit von dem Qualitätsfaktor (siehe Tabelle unten)
- Zyklus # 11-: Schaltet auf Gelb 8 bis 9 Tage vor dem frühesten zweiten Tag der bisher gemessenen Temperaturanstiege in Abhängigkeit von dem Qualitätsfaktor (siehe Tabelle unten)

Rote Statusleuchte - FRUCHTBAR - wird einen Tag nach gelber Statusleuchte angezeigt

### Besonders riskante Tage (Qx = Qualitätsfaktor der Frau)

| **Zyklus #** | **Q7** | **Q6** | **Q5** | **Q4** | **Q3** | **Q2** | **Q1** | **Q0** | **Min.** | **Max.** |
|---|---|---|---|---|---|---|---|---|---|---|
| **1-5** | - | - | - | - | k.A. | - | - | - | - | - |
| **6** | 0 | 1 | 1 | 2 | 2 | 3 | 3 | 3 | **0** | **3** |
| **7** | 0 | 0 | 1 | 1 | 2 | 2 | 3 | 3 | **0** | **3** |
| **8** | 0 | 0 | 0 | 1 | 1 | 2 | 2 | 3 | **0** | **2** |
| **9** | 0 | 0 | 0 | 0 | 1 | 1 | 2 | 2 | **0** | **2** |
| **10** | 0 | 0 | 0 | 0 | 0 | 1 | 1 | 2 | **0** | **2** |
| **11** | 0 | 0 | 0 | 0 | 0 | 0 | 1 | 1 | **0** | **1** |
| **12-** | 0 | 0 | 0 | 0 | 0 | 0 | 0 | 1 | **0** | **1** |

Die äußerste linke Spalte bezeichnet Tag 0, die äußerste rechte Spalte bezeichnet Tag 28. Ovulation am 14. Tag und Temperaturanstieg am 16. Tag.

**Fig. 3** zeigt beispielsweise den Verlauf der Zyklusabschnitte in der Anzeigevorichtung des erfindungsgemäßen Geräts.

Übergang Nr. 1 ist der erste Teil des Zyklus zu Zyklusbeginn. Es werden beispielsweise drei mögliche unfruchtbare Tage angezeigt. Dann erfolgt eine Änderung von möglicherweise unfruchtbar Tagen zu möglicherweise fruchtbar Tagen, zum Beispiel während der Dauer von zehn Tagen. Übergang Nr. 2 ist der Teil des Zyklus, während dem eine Änderung von möglicherweise fruchtbaren Tagen zu möglicherweise unfruchtbaren Tagen nach der Ovulatitin erfolgt. Beispielsweise kann die Dauer der angezeigten möglichen unfruchtbaren Tagen 12 Tage sein.

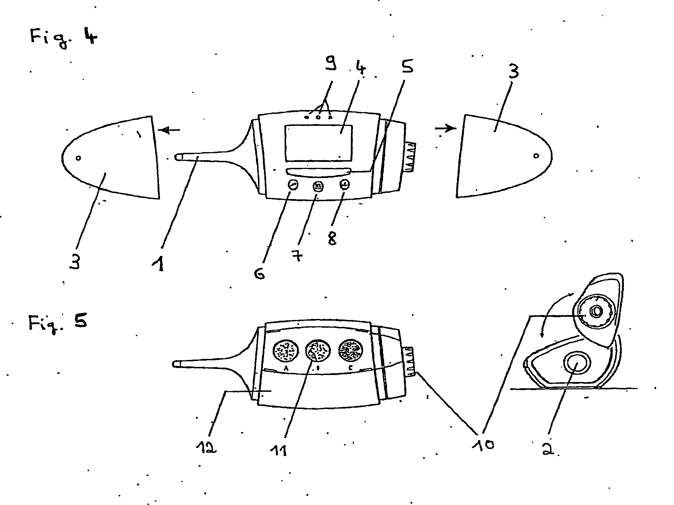

**Fig. 4** zeigt die Aufsicht auf eine bevorzugte Ausführungsform des erfindungsgemäßen prozessgesteuerten Mini Computers zur Ovulationsbestimmung bzw. Familienplanung. Dabei werden vorzugsweise 3 bewährte Testmethoden der natürlichen Familienplanung (NFP) - Kalender-, Basaltemperatur- und Speichelmethode sinnvoll kombiniert und mit einer algorithmischen Software ausgewertet. Das Resultat wird auf dem Display (4) angezeigt bzw. ist durch Betätigung der Funktionstaste (5) jederzeit abrufbar. Mittels der Taste für Zykluseingabe (7) wird der erste Tag der letzten Regelblutung eingegeben, damit das Gerät eine erste Berechnungsgrundlage nach der Kalendermethode hat, um die Anwenderin einer Zyklusgruppe zuzuordnen. Es besteht sogar die Möglichkeit diese Eingabe drei bis fünf Tage nach Eintritt der Regel zu bestätigen. Spätestens nach dem 5. Zyklus, bevorzugt nach dem 3. Zyklus hat das Gerät seine maximale Effizienz erreicht, so dass das Gerät umso präziser wird, je länger es benützt wird und ein Pearl-Index von 0,5 - 2 erreicht wird. Da die Messungen vorzugsweise morgens vor dem Aufstehen durchgeführt werden sollen, jedenfalls regelmäßig zu gleicher Uhrzeit, umfasst das Gerät einen eingebauten Wecker bzw. Alarm, der durch Betätigung der Funktionstaste (5) auf die gewünschte Uhrzeit eingestellt wird. Zur Messung der Basaltemperatur wird die Taste für Temperaturmessung (6) gedrückt, die Schutzkappe (3) über dem Temperaturfühler (1) wird entfernt und der Temperaturfühler (1) wird für einige Sekunden unter eine Zungenhälfte geführt bis die Messung beendet ist. Das Ende der Messung wird durch ein optisches oder akustisches Signal, bevorzugt einen Bestätigungston, angezeigt. Der gemessene Wert ist auf dem Display (4) ablesbar und durch drücken der Funktionstaste (5) auf dem Rechner speicherbar. Anschließend leuchtet die Statutsleuchte (9) auf, die Information über die Zyklusphase gibt. Die Statusleuchte (9) besteht bevorzugt aus drei Leuchten, wobei grün anzeigt, dass keine Empfängnis möglich ist, gelb eine Übergangsphase mit unsicherer Empfängnis anzeigt und rot die Ovulationsphase als die sicherste Zeit für Empfängnis angibt.

5-10 mal, bevorzugt 6-8 mal pro Zyklus fordert das Gerät mittels der Aufforderungseinrichtung zur Durchführung einer zweiten Messmethode auf, vorzugsweise zur Speicheltestmethode. Dazu ist im Display (4) eine Meldung wie z.B. "Bitte Speicheltest machen", zu lesen, die von einem Signal, z.B. einem Warnton begleitet wird. Zur Messung des Speichels, bevorzugt des Morgenspeichels, wird die Taste für Speicheltest (8) gedrückt und die Schutzkappe (3) über dem Speichelträger (2); siehe Figur 5)) entfernt. Die Oberfläche des Trägers (2) wird kurz mit einem Tuch gereinigt und ein wenig Speichel, bevorzugt Morgenspeichel, aus dem Mund auf den Träger (2) aufgetragen. Vorzugsweise nach dem Trocknen des Speichels auf dem Träger (2) wird der im Gerät hinterleuchtete Testspeichel dann mit Referenzbildern verglichen, die bevorzugt auf der Rückseite des Messgeräts wie bei Fig. 5 gezeigt, angebracht sind, um die Phase des Zyklus anhand dieser Bilder zu bestimmen. Die gemessenen Daten werden durch Betätigung der Funktionstaste (5) in dem Gerät gespeichert.

**Fig. 5** zeigt die bevorzugte Ausführungsform der Rückseite des Messgeräts, die vorzugsweise 3-5 Vergleichsbilder (11) für die Kristallstruktur des Speichels aufweist. Der Speichel der Anwenderin, der sich auf dem Speichelträger (2) befindet, wird durch ein einstellbares Okular (10) betrachtet und mit den Bildern (11) verglichen. Die Ergebnisse dieser Messung werden durch drücken der Funktionstaste (5), die sich auf der anderen Seite des Geräts (Fig. 4) befindet, in dem Gerät gespeichert.

Weiterhin umfasst diese Seite des Geräts einen Anschluss und / oder ein Fach (12) für die Energieversorgung des Geräts. Die Energieversorgung während des Betriebs erfolgt mittels eines Netzsteckers, eines Akkus oder einer Batterie. Der Ladungszustand des Geräts wird über eine Leuchtdiode angezeigt.

**Tabelle 1** zeigt beispielhaft die Ableitung eines Indikators für die Regelmäßigkeit der Zyklusdauer zur Bestimmung des Übergangs Nr. 2.

| **Gebrauch des Punkte-Systems** | |
|---|---|
| Zyklusbeginn . | Punkte = 0 |
| Erster Tag, an dem ein Temperaturanstieg festgestellt wird | Punkte = 1 |
| Zwei Tage Temperaturanstieg hintereinander | Punkte = 2 |
| Nach drei Tagen Temperaturanstieg | Punkte = 3 |
| Nach vier Tagen Temperaturanstieg usw. | Punkte = 4 |

### YellowStartPoints (Ausganspunkte in Gelb) - "Belohnungs-Bestrafungs-System"

| **Zeitabhängiges "Belohnungs-Bestrafungs-System" der YellowStartPoints** | | |
|---|---|---|
| Zyklus # 1-4 | "Bestrafung" + 1 Punkt | YellowStartPoints + 1 |
| Zyklus # 5- | "Belohnung-Bestrafungs-System" in Abhängigkeit von Sigma | YellowStartPoints + x |
| | Ovulations-Zeit-Sigma für GUT | x = -1 |
| | Ovulations-Zeit-Sigma für NORMAL | x = 0 |
| | Ovulations-Zeit-Sigma für SCHLECHT | x = +1 |

| "**Speichelbestrafung"** | | |
|---|---|---|
| Zyklus # 1-2 | "Bestrafung" + 2 Punkte | YellowStartPoints + 2 |
| Zyklus # 3- | "Bestrafung" 0 oder + 2 Punkte | YellowStartPoints + y |
| | Muster "A" | y = 0 |
| | Muster "B" | y = 0 |
| | Muster "C" | y = +2 |

### Zusammenfassung der YellowStartPoints (Ausgangspunkte in Gelb)

| **Zyklus #** | **Standardeinstellung** | **Zeitpunkt** | **Speichel** | **Min.** | **Max.** |
|---|---|---|---|---|---|
| 1 | 3 | +1 | +2 | **6** | **6** |
| 2 | 3 | +1 | +2 | **6** | **6** |
| 3 | 3 | +1 | 0/+2 | **4** | **6** |
| 4 | 3 | +1 | 0/+2 | **4** | **6** |
| 5- | 3 | -1/0/+1 | 0/+2 | **2** | **6** |

### YellowStartPoints (ab wann der Beginn der unfruchtbaren Tage angezeigt wird)

| | |
|---|---|
| "Belohnungs-Bestrafungs-System" | YellowStartPoints = 2 bis 6 |

### GreenStartPoints (Ausgangspunkte in Grün; ab wann der Beginn der unfruchtbaren Tage angezeigt wird)

| | |
|---|---|
| GreenStartPoints | YellowStartPoints + 1 |

### Änderung der Statusleuchte

Änderung zu UNFRUCHTBAR (gelbe Statusleuchte) wenn Punkte = YellowStartPoints. UNFRUCHTBAR (grüne Statusleuchte) einen Tag nach gelber STATUSLEUCHTE.

### Liste der Bezugszeichen:

### Fig. 1 Blockdiagramm-Darstellung des erfindungsgemäßen Geräts

- (1): Mittel zur Bestimmung der Basaltemperatur
- (2): Speichelträger
- (4): Anzeigevorrichtung
- (5): Funktionstaste
- (6): Mittel zur Eingabe der Temperatur
- (7): Mittel zur Eingabe des ersten Tages des Zyklus
- (8): Mittel zur Eingabe der Beschaffenheit des Speichels
- (9): Statusleuchten
- (10): Okular
- (13): Vorrichtung zur Verarbeitung der durch die Mittel (1, 7, 8) bereitgestellten Daten
- (14): Speichervorrichtung

### Fig. 4 Vorderseite des Messgeräts

- (1): Mittel zur Bestimmung der Basaltemperatur
- (3): Schutzkappen
- (4): Anzeigevorrichtung
- (5): Funktionstaste
- (6): Mittel zur Eingabe der Temperatur
- (7): Mittel zur Eingabe des ersten Tages des Zyklus
- (8): Mittel zur Eingabe der Beschaffenheit des Speichels
- (9): Statusleuchten

### Fig. 5 Rückseite des Messgeräts

- (2): Speichelträger
- (10): schwengbares, einstellbares Okular
- (11): Vergleichsbildern für den Speichelschleim
- (12): Batteriedeckel

## Patentansprüche

1. Gerät zur Familienplanung und/oder Verhütung, mit
einem Mittel (1) zur Bestimmung der Basaltemperatur,
einem Mittel (7) zur Eingabe des ersten Tages der Periode,
einem Mittel (8) zur Eingabe der Beschaffenheit des Speichels,
einer Vorrichtung zur Verarbeitung der durch die Mittel (1, 7, 8) bereitgestellten Daten (13),
einer Speichervorrichtung (14) zum Speichern von zumindest einigen der bereitgestellten Daten und
einer Anzeigeeinrichtung (4),
wobei die Vorrichtung zur Verarbeitung der Daten derart ausgestaltet ist, dass in Abhängigkeit von zumindest einigen der bereitgestellten Daten eines jeden Mittels eine Aussage zur Fruchtbarkeit an einem beliebigen Tag ermittelt wird, die über die Anzeigeeinrichtung anzeigbar ist und die Daten beim Ermitteln der Aussage zur Fruchtbarkeit unterschiedlich gewichtet werden.

2. Gerät nach Anspruch 1, **dadurch gekennzeichnet, dass** die Gewichtung der Daten in Abhängigkeit der vormals bereitgestellten, gespeicherten Daten variiert.

3. Gerät nach Anspruch 1 oder 2, **dadurch gekennzeichnet,**
**dass** die Aussage zur Fruchtbarkeit in stärkerem Maße von den durch das Mittel (1) zur Bestimmung der Basaltemperatur bereitgestellten Daten abhängt, als von den durch das Mittel (7) zur Eingabe des ersten Tages des Zyklus bereitgestellten Daten und dass die durch das Mittel (7) zur Eingabe des ersten Tages der Periode bereitgestellten Daten ihrerseits einen größeren Einfluss auf die Aussage zur Fruchtbarkeit haben, als die durch das Mittel (8) zur Eingabe der Beschaffenheit des Speichels bereitgestellten Daten.

4. Gerät nach einem der Ansprüche 1 bis 3, **dadurch gekennzeichnet,**
**dass** die Gewichtung der durch das Mittel (1) zur Bestimmung der Basaltemperatur bereitgestellten Daten vorzugsweise bei 50-90%, besonders bevorzugt bei 60-80%, die Gewichtung der durch das Mittel (7) zur Eingabe des ersten Tages der Periode bereitgestellten Daten, vorzugsweise bei 5-35%, besonders bevorzugt bei 10-30%, und die Gewichtung der durch das Mittel (8) zur Eingabe der Beschaffenheit des Speichels bereitgestellten Daten vorzugsweise bei 2-20%, besonders bevorzugt bei 5-15%, liegt.

5. Gerät nach einem der vorstehenden Ansprüche, **dadurch gekennzeichnet, dass** die Vorrichtung zur Verarbeitung der Daten derart ausgestaltet ist, dass die Aussage zur Fruchtbarkeit zwischen möglichen fruchtbaren und unfruchtbaren Tagen unterscheidet und dass die möglichen fruchtbaren und unfruchtbaren Tage unterschiedlich optisch dargestellt werden.

6. Gerät nach Anspruch 5, **dadurch gekennzeichnet, dass** die Anzeigeeinrichtung die möglichen fruchtbaren Tage in roter Farbe und die möglichen unfruchtbaren Tage in grüner Farbe signalisiert.

7. Gerät nach Anspruch 5 oder 6, **dadurch gekennzeichnet, dass** in der Aussage zur Fruchtbarkeit auch Übergangstage vorgesehen sind, an denen nicht zwischen möglichen fruchtbaren und unfruchtbaren Tagen unterschieden wird,
wobei die Übergangstage von den möglichen fruchtbaren und unfruchtbaren Tagen optisch unterschiedlich dargestellt werden.

8. Gerät nach Anspruch 7, **dadurch gekennzeichnet, dass** die Anzeigeeinrichtung die Übergangstage in gelber Farbe signalisiert.

9. Gerät nach einem der Ansprüche 5 bis 8, **gekennzeichnet durch** ein Mittel zum Erkennen eines Temperaturanstieges aus den **durch** das Mittel (1) zur Bestimmung der Basaltemperatur bereitgestellten Daten, wobei beim Ermitteln der Aussage zur Fruchtbarkeit ein erster Periodenabschnitt zu Beginn einer Periode und ein zweiter Periodenabschnitt, der nach dem erkannten Temperaturanstieg liegt, unterschieden wird.

10. Gerät nach Anspruch 9, **dadurch gekennzeichnet, dass** bis zu einem ersten Zeitpunkt, beispielsweise dem Ende der zweiten durch das Gerät registrierten Zyklus, in der Aussage zur Fruchtbarkeit im zweiten Zyklusabschnitt die Anzahl der möglichen fruchtbaren Tage vorgegeben ist.

11. Gerät nach Anspruch 10, **dadurch gekennzeichnet, dass** nach dem ersten Zeitpunkt in der Aussage zur Fruchtbarkeit im zweiten Zyklusabschnitt die Anzahl von möglichen fruchtbaren Tagen in Abhängigkeit einer Auswertung gespeicherter Daten bestimmt wird.

12. Gerät nach Anspruch 11, **dadurch gekennzeichnet, dass** aus den gespeicherten Daten ein Indikator für die Regelmäßigkeit der Zyklusdauer bzw. des Zyklusverlaufs abgeleitet wird und dass in der Aussage zur Fruchtbarkeit im zweiten Zyklusabschnitt die Anzahl der möglichen unfruchtbaren Tage in Abhängigkeit dieses Indikator und/oder der Anzahl der durch das Gerät registrierten Zyklen bestimmt wird.

13. Gerät nach einem der Ansprüche 9 bis 12, **dadurch gekennzeichnet, dass** in der Aussage zur Fruchtbarkeit im zweiten Periodenabschnitt die Anzahl von möglichen fruchtbaren Tagen in Abhängigkeit der durch das Mittel (8) zur Eingabe der Beschaffenheit des Speichels bereitgestellten Daten bestimmt bzw. beeinflusst wird.

14. Gerät nach einem der Ansprüche 9 bis 13, **dadurch gekennzeichnet, dass** bis zu einem zweiten Zeitpunkt, beispielsweise dem Ende des fünften durch das Gerät registrierten Zyklus, in der Aussage zur Fruchtbarkeit im ersten Zyklusabschnitt die Anzahl der möglichen unfruchtbaren Tage vorgegeben ist.

15. Gerät nach Anspruch 14, **dadurch gekennzeichnet, dass** nach dem zweiten Zeitpunkt in der Aussage zur Fruchtbarkeit im ersten Zyklusabschnitt die Anzahl der möglichen unfruchtbaren Tage in Abhängigkeit einer Auswertung gespeicherter Daten bestimmt wird.

16. Gerät nach Anspruch 15, **dadurch gekennzeichnet, dass** aus den gespeicherter Daten ein Qualitätsfaktor abgeleitet wird und dass nach dem zweiten Zeitpunkt in der Aussage zur Fruchtbarkeit im ersten Zyklusabschnitt die Anzahl von möglichen unfruchtbaren Tagen in Abhängigkeit des Qualitätsfaktors und/oder der Anzahl der durch das Gerät registrierter Perioden bestimmt wird in Abhängigkeit zunächst einiger Daten des Mittels (7) zur Eingabe des ersten Tages des Zyklus.

17. Gerät nach einem der vorstehenden Ansprüche, **gekennzeichnet durch** eine Zeitmessvorrichtung und eine Aufforderungseinrichtung zur Aufforderung einer Benutzerin zur Eingabe von Daten nach Verstreichen einer vorbestimmten Zeitspanne.

18. Gerät nach Anspruch 17, **dadurch gekennzeichnet, dass** die Aufforderungseinrichtung derart gestaltet ist, dass eine Eingabe zur Beschaffenheit des Speichels vorzugsweise 5-10 mal, besonders bevorzugt 6-8 mal pro Periode gefordert wird.

19. Gerät nach einem der vorstehenden Ansprüche, derart ausgestaltet, dass Temperaturschwankungen, die nicht durch die Periode hervorgerufen sind, sondern auf anderen Faktoren, wie z.B. einer Erkältung oder Migräne beruhen, erkannt und daraufhin die entsprechenden Daten bei der Bestimmung der möglichen fruchtbaren bzw. unfruchtbaren Tage verworfen werden.

20. Gerät nach einem der vorstehenden Ansprüche, derart ausgestaltet, dass ein Zyklus und insbesondere deren Dauer bzw. Verlauf erkannt wird.

## Claims

1. A device for family planning and/or contraception, whereby the device has
a means (1) for determining basal temperature,
a means (7) for input of the first day of the cycle,
a means (8) for input of the property of the saliva,
a device for processing the data (13) made available by the means (1, 7, 8),
a memory device (14) for storing at least some of the data made available and
a display device (4),
whereby the device for processing the data is designed such that a conclusion regarding the woman's fertility on any given day is determined as a function of at least some of the data made available by each means and this conclusion can be displayed via the display device and the data are weighted differently in determining the conclusion regarding the woman's fertility.

2. The device according to Claim 1, **characterized in that** the weighting of the data can be varied as a function of the stored data formerly made available.

3. The device according to claims 1 or 2, **characterized in that** the conclusion regarding the woman's fertility depends on the data made available by the means (1) for determining the basal temperature to a greater extent than on the data made available by the means (7) for input of the first day of the woman's cycle, and the data made available by the means (7) for input of the first day of the woman's cycle in turn has a greater influence on the conclusion regarding her fertility than does the data made available by the means (8) for input of the properties of the saliva.

4. The device according to one of the Claims 1 to 3, **characterized in that** the weighting made available by the means (1) for determining the basal temperature preferably amounts to 50-90 %, especially preferably 60-80 %; the weighting of the data made available by the means (7) for input of the first day of the woman's cycle preferably amounts to 5-35 %, especially preferably 10-30 %, and weighting of the data made available by the means (8) for input of the properties of the saliva preferably amounts to 2-20 %, especially preferably 5-15 %.

5. The device according to one of the previous Claims, **characterized in that** the device is equipped for processing data, such that the conclusion regarding a woman's fertility differentiates between possible fertile and infertile days and that the possible fertile and infertile days are displayed visually in different ways.

6. The device according to Claim 5, **characterized in that** the display device shows the possible fertile days in red and the possible infertile days in green.

7. The device according to Claims 5 or 6, **characterized in that** transition days are also provided in the conclusion regarding the woman's fertility; these are days when no distinction is made between possible fertile days and possible infertile days, and the transition days are displayed differently visually than the possible fertile days and infertile days.

8. The device according to Claim 7, **characterized in that** the display device displays the transition days in yellow.

9. The device according to one of the Claims 5 to 8, **characterized by** a means for recognizing an increase in temperature from the data made available by the means (1) for determining the basal temperature, whereby in determining the conclusion regarding the woman's fertility, a distinction is made between a first phase of the cycle at the beginning of the cycle and a second phase of the cycle after the rise in temperature is detected.

10. The device according to Claim 9, **characterized in that** up to a first point in time, for example, the end of the second cycle registered by the device, the number of possible fertile days is preselected in the conclusion regarding the woman's fertility in the second phase of the cycle.

11. The device according to Claim 10, **characterized in that** after the first point in time, the number of possible fertile days is determined as a function of an analysis of stored data in the conclusion regarding the woman's fertility in the second phase of the cycle.

12. The device according to Claim 11, **characterized in that** an indicator for the regularity of the duration of the cycle and/or the course of the cycle is derived from the stored data, and the number of possible infertile days is determined as a function of this indicator and/or the number of cycles registered by the device is determined in the conclusion regarding the woman's fertility in the second phase of the cycle.

13. The device according to one of the Claims 9 to 12, **characterized in that** the number of possible fertile days is determined and/or influenced as a function of the data made available by the means (8) for input of the properties of the saliva in the conclusion regarding fertility in the second phase of the cycle.

14. The device according to one of the Claims 9 to 13, **characterized in that** up to a second point in time, e.g., the end of the fifth cycle registered by the device, the number of possible infertile days is preselected in the conclusion regarding the woman's fertility in the first phase of the cycle.

15. The device according to Claim 14, **characterized in that** after the second point in time, the number of possible infertile days is determined as a function of an analysis of stored data in the conclusion regarding the woman's fertility in the first phase of the cycle.

16. The device according to Claim 15, **characterized in that** a quality factor is derived from the stored data, and after the second point in time, in the conclusion regarding the woman's fertility in the first phase of the cycle, the number of possible infertile days is determined as a function of the quality factor and/or the number of periods registered by the device is determined as a function of some data from the means (7) for input of the first day of the cycle.

17. The device according to one of the previous Claims, **characterized by** a time measuring device and an interactive device for instructing a user to input data after a predetermined period of time has elapsed.

18. The device according to Claim 17, **characterized in that** the interactive device is designed so that an input of the properties of the saliva is instructed preferably 5-10 times per cycle, especially preferably 6-8 times per cycle.

19. The device according to one of the previous claims, designed so that fluctuations in temperature that are not due to the cycle but instead are caused by other factors such as a cold or a migraine are recognized and then to discard this data when determining the possible fertile and/or infertile days.

20. The device according to one of the previous claims, designed so that a cycle and in particular the duration and/or the course of the cycle are recognized.

## Revendications

1. Outil de planning familial et/ou de prévention de la grossesse, qui présente :
un moyen (1) de détermination de la température basale,
un moyen (7) d'introduction du premier jour du cycle,
un moyen (8) d'introduction de la qualité de la salive,
un dispositif de traitement des données (13) délivrées par les moyens (1, 7, 8),
un dispositif de mémoire (14) qui conserve en mémoire au moins certaines des données délivrées et
un dispositif d'affichage (4),
le dispositif de traitement des données étant configuré de telle sorte qu'en fonction d'au moins certaines des données préparées par chacun des moyens, une conclusion sur la fertilité à un jour quelconque est déterminée et peut être affichée par le dispositif d'affichage, les données étant pondérées différemment lors de la détermination de la conclusion sur la fertilité.

2. Appareil selon la revendication 1, **caractérisé en ce que** la pondération des données varie en fonction des données conservées en mémoire et délivrées précédemment.

3. Appareil selon les revendications 1 ou 2, **caractérisé en ce que** la conclusion sur la fertilité dépend dans une mesure plus importante des données délivrées par le moyen (1) de détermination de la température basale que des données délivrées par le moyen (7) d'introduction du premier jour du cycle, et **en ce que** les données délivrées par le moyen (7) d'introduction du premier jour du cycle ont une plus grande influence sur la conclusion sur la fertilité que les données délivrées par le moyen (8) d'introduction de la qualité de la salive.

4. Appareil selon l'une des revendications 1 à 3, **caractérisé en ce que** la pondération des données délivrées par le moyen (1) de détermination de la température basale est de préférence de 50 à 90 % et de façon particulièrement préférable de 60 à 80 %, la pondération des données délivrées par le moyen (7) d'introduction du premier jour du cycle est de préférence de 5 à 35 % et de façon particulièrement préférable de 10 à 30 % et la pondération des données délivrées par le moyen (8) d'introduction de la qualité de la salive est de préférence de 2 à 20 % et de façon particulièrement préférable de 5 à 15 %.

5. Appareil selon l'une des revendications précédentes, **caractérisé en ce que** le dispositif de traitement des données est configuré de telle sorte que la conclusion sur la fertilité effectue une distinction entre des jours éventuellement fertiles et des jours éventuellement infertiles et **en ce que** les jours éventuellement fertiles et les jours éventuellement infertiles sont présentés visuellement de manière différente.

6. Appareil selon la revendication 5, **caractérisé en ce que** le dispositif d'affichage signale les jours éventuellement fertiles en couleur rouge et les jours éventuellement infertiles en couleur verte.

7. Appareil selon les revendications 5 ou 6, **caractérisé en ce que** dans les conclusions sur la fertilité, on prévoit également des jours de transition pour lesquels aucune distinction n'est faite entre des jours éventuellement fertiles et des jours éventuellement infertiles, les jours de transition étant présentés de manière visuellement différente des jours éventuellement fertiles et des jours éventuellement infertiles.

8. Appareil selon la revendication 7, **caractérisé en ce que** le dispositif d'affichage signale les jours de transition en couleur jaune.

9. Appareil selon l'une des revendications 5 à 8, **caractérisé par** un moyen de détection d'une augmentation de température à partir des données délivrées par le moyen (1) de détermination de la température basale, et en ce que lors de la détermination de la conclusion sur la fertilité, une distinction est faite entre une première partie de cycle au début d'un cycle et une deuxième partie de cycle située après l'augmentation de température qui a été détectée.

10. Appareil selon la revendication 9, **caractérisé en ce que** jusqu'à un premier moment, par exemple la fin du deuxième cycle enregistré par l'appareil, le nombre des jours éventuellement fertiles est prédéterminé dans la conclusion sur la fertilité de la deuxième partie du cycle.

11. Appareil selon la revendication 10, **caractérisé en ce qu'**après le premier moment, on détermine dans la conclusion sur la fertilité de la deuxième partie du cycle le nombre de jours éventuellement fertiles sur base d'une évaluation des données conservées en mémoire.

12. Appareil selon la revendication 11, **caractérisé en ce qu'**à partir des données conservées en mémoire, on déduit un indicateur de régularité de la durée du cycle ou de l'évolution du cycle et **en ce que** dans la conclusion sur la fertilité dans la deuxième partie du cycle, on détermine le nombre des jours éventuellement infertiles en fonction de cet indicateur et/ou du nombre des cycles enregistrés par l'appareil.

13. Appareil selon l'une des revendications 9 à 12, **caractérisé en ce que** dans la conclusion sur la fertilité au cours de la deuxième partie du cycle, le nombre des jours éventuellement fertiles est déterminé ou influencé par les données délivrées par le moyen (8) d'introduction de la qualité de la salive.

14. Appareil selon l'une des revendications 9 à 13, **caractérisé en ce que** jusqu'à un deuxième moment, par exemple la fin du cinquième cycle enregistré par l'appareil, le nombre des jours éventuellement infertiles est prédéterminé dans la conclusion sur la fertilité au cours de la première partie du cycle.

15. Appareil selon la revendication 14, **caractérisé en ce qu'**après le deuxième moment, le nombre des jours éventuellement infertiles est déterminé en fonction d'une évaluation des données conservées en mémoire dans la conclusion sur la fertilité au cours de la première partie du cycle.

16. Appareil selon la revendication 15, **caractérisé en ce que** l'on déduit des données conservées en mémoire un facteur de qualité et **en ce qu'**après le deuxième moment, le nombre des jours éventuellement infertiles est déterminé en fonction du facteur de qualité et/ou du nombre des cycles enregistrés par l'appareil lors de la conclusion sur la fertilité au cours de la première partie du cycle, en fonction d'abord de certaines données du moyen (7) d'introduction du premier jour du cycle.

17. Appareil selon l'une des revendications précédentes, **caractérisé par** un dispositif de mesure du temps et un dispositif d'invitation qui invite une utilisatrice à introduire des données après qu'une durée prédéterminée s'est écoulée.

18. Appareil selon la revendication 17, **caractérisé en ce que** le dispositif d'invitation est configuré de telle sorte qu'une introduction concernant la qualité de la salive est demandée de préférence 5 à 10 fois et de façon particulièrement préférable 6 à 8 fois par cycle.

19. Appareil selon l'une des revendications précédentes, **caractérisé en ce que** des variations de température qui ne sont pas provoquées par le cycle mais par d'autres facteurs, par exemple un refroidissement ou une migraine, sont détectées et qu'ensuite les données correspondantes sont rejetées lors de la détermination des jours éventuellement fertiles et des jours éventuellement infertiles.

20. Appareil selon l'une des revendications précédentes, configuré de manière à détecter un cycle et en particulier sa durée ou son évolution.
